# EUROPEAN PATENT SPECIFICATION

(11) **EP 3 125 977 B1**
(45) Date of publication and mention of the grant of the patent: **29.07.2020**
(21) Application number: 15714491.6
(22) Date of filing: 02.04.2015
(51) Int. Cl.: A61M 11/00, A61M 15/00

(54) **DRY POWDER INHALER**
TROCKENPULVERINHALATOR
INHALATEUR DE POUDRE SÈCHE

(30) Priority: 03.04.2014 SE 1450410
(43) Date of publication of application: 08.02.2017
(73) Proprietor: Iconovo AB, 223 63 Lund (SE)
(72) Inventor: LASTOW, Orest, S-247 45 Torna Hällestad (SE); ARVIDSSON, Lars, S-247 51 Dalby (SE)
(74) Representative: Ström & Gulliksson AB
(86) International application number: PCT/EP2015/057315
(87) International publication number: WO 2015/150518

(56) References cited:
- EP-A1- 0 387 222
- EP-A1- 1 488 819
- EP-A2- 1 208 863
- WO-A1-2011/059953
- WO-A1-2011/059968
- WO-A2-2006/037636
- WO-A2-2009/009013
- WO-A2-2009/145673
- DE-A1-102007 017 725
- DE-C1- 4 415 462
- GB-A- 2 460 281
- JP-A- 2011 212 269
- US-A- 4 805 811

## Description

### Field of the Invention

This invention pertains in general to the field of medicament inhalers, and more particularly to dry powder inhalers. The inhaler comprises at least one air inlet and at least one air outlet, and a reservoir for housing a dry powder drug, the inhaler having an airflow from the at least one air inlet to the at least one outlet during inhalation by a user at said at least one outlet, to deliver said dry powder drug during inhalation by the user.

### Background of the Invention

In the pharmaceutical field, with respect to treatment of respiratory and/or other diseases, inhalers have been widely used. Numerous drugs, medications and other substances are inhaled into the lungs for rapid absorption in the blood stream and for local action in the lung with such inhalers.

Inhaled drugs fall into two main categories, in form of liquids, including suspensions, and powders. The choice of category depends on the characteristics of the drugs, medications, etc., to be inhaled.

The most common type of inhaler is the pressurized metered-dose inhaler. In this type of inhaler medication is most commonly stored in solution in a pressurized canister that contains a propellant, although it may also be a suspension. The canister is attached to a plastic, hand-operated actuator. On activation, the metered-dose inhaler releases a fixed dose of medication in aerosol form.

Another kind of inhaler is a nebulizer, which supply medication as an aerosol created from an aqueous formulation.

The kind referred to herein is yet another type, in form of a dry powder inhaler. A dry powder inhaler releases a pre-metered, capsuled, dose or a device-metered dose of powdered medication that is inhaled through the inhaler. Inhalers with device-metered dose of powdered medication is normally inhalers with medication reservoir, containing powdered medication, from which metered doses are withdrawn through the use of different dose metering arrangements, said doses then being inhaled.

Dry powder inhalers need to deliver a particle size that is predominantly below 5 microns, and preferably between 1 micron and 3.3 microns, for maximum effectiveness. Such small particles are, however, very cohesive due to high surface energy. Agglomeration may be worsened by moisture, and when the medication comprises more than one active substance, since the different active substances may have such properties as to form agglomerations with each other or with pharmaceutical carriers etc. Agglomeration of small particles is a problem which results in the active particles leaving the inhaler as large agglomerates.

EP0237507 discloses one such powder inhaler with a device metered dose, comprising a medicament chamber, a dosing mechanism, and a flow path from an air inlet to an air/medicament outlet. In the flow path deflectors are arranged, to increase deaggregation of medicament. However, this device is limited to medicaments having one active substance or active substances that are compatible with each other during storing. Additionally, medicament will accumulate at the deflectors, decreasing uniformity of dosage.

WO2011/059953A1 relates to dry powder inhalers and drug products and, more particularly to polyflux colliders useful for de-agglomerating dry powder in dry powder dispensers. Various embodiments provide drug products, dry powder inhalers and polyflux collider arrangement. With various embodiments of the present invention, a polyflux collider is provided which utilizes colliding streams of dry powder to provide desirable de-agglomerating capability for dry powder dispensers.

WO2009/145673A2 relates to powder inhalators for easy use and for simultaneously introducing two active components into a patient lungs. The powder inhalator comprises a body which can be covered with a jacket, is provided with openings which are used for air intake and aerosol exit and are interconnected by an air duct comprising two opposite accelerating channels, each of which is connected to a disperser. The powder inhalator also comprises a powder container and a dozer in the form of a movable plate with two measuring orifices shifted in relation to each other at a rotation angle of 90°. The container consists of two insulated sections, in that each section is provided with a powder supplying orifice, in that the movable plate of the dozer is additionally provided with two measuring orifices which are diametrically opposite in such a way that when the movable plate of the doser is turned at an angle of 90° in one of the directions for supplying a powder portion or when said plate is turned at an angle of 90° in the opposite direction for supplying the next powder portion, the two of the measuring orifices are placed under the orifices of the two section of the container and the two other measuring orifices are placed at the bases of the two accelerating channels.

WO2011/059968A1 discloses drug products and dry powder inhalers and powder dispensers with multiple reservoirs. Several embodiments provide a drug product comprising a dry powder inhaler and at least one dose of at least one active pharmaceutical agent; wherein the dry powder inhaler comprises at least two reservoirs. Other embodiments provide for a powder dispenser which includes a first powder reservoir having at least one first opening, and a second powder reservoir having at least one second outlet opening, the second outlet opening being spaced from the first outlet opening.

An additional problem of the prior art inhalers is that they are either suitable for micronized formulations or carrier based formulations - never both or combinations of these.

In view of these drawbacks and limitations of the prior art, what is needed is a dry powder inhaler device in which effective and satisfactory dispersion of the dry powder is obtained, which inhaler can administer medicament comprising substances which are incompatible in mixture, and an inhaler with increased deaggregation and a more uniform dosage, as well as an inhaler which is suitable for both micronized formulations and/or carrier based formulations.

### Summary of the Invention

Accordingly, the present invention preferably seeks to mitigate, alleviate or eliminate one or more of the above-identified deficiencies in the art and disadvantages singly or in any combination and solves at least the above mentioned problems by providing a dry powder drug inhaler according to claim 1. The dependent claims define preferred embodiments of the invention.

A method for expelling and mixing a medicament dose from an inhaler is also described, but is not part of the claimed invention.

### Brief Description of the Drawings

These and other aspects, features and advantages of which the invention is capable will be apparent and elucidated from the following description of embodiments of the present invention, reference being made to the accompanying drawings, in which
Fig. 1 is a cross sectional view along a longitudinal axis of an inhaler according to one embodiment in the dose administering position of the present invention;
Fig. 2 is a perspective and cross sectional view of an inhaler according to one embodiment of the present invention;
Fig. 3 is a perspective and cross sectional view of an inhaler according to one embodiment of the present invention, focusing on arrangement of openings in dose disc;
Fig. 4 is a perspective and cross sectional view of an inhaler according to one embodiment of the present invention, focusing on arrangement of openings in dose disc;
Fig. 5 is a perspective and cross sectional view of an inhaler according to one embodiment of the present invention, focusing on arrangement of medicament scraper over opening in dose disc;
Fig. 6 is a perspective and cross sectional view of an inhaler according to one embodiment of the present invention, focusing on arrangement of medicament scraper and medicament in opening;
Fig. 7 is a cross sectional view along a longitudinal axis of an inhaler according to an embodiment of the present invention, focusing on arrangement of medicament scraper over opening in dose disc; and
Fig. 8 is a cross sectional view along a longitudinal axis of an inhaler according to one embodiment, wherein the air and air/medicament flows through this inhaler is disclosed.

### Description of embodiments

The following description focuses on an embodiment of the present invention being a dry powder drug inhaler with more than one medicament reservoir, such as two medicament containers.

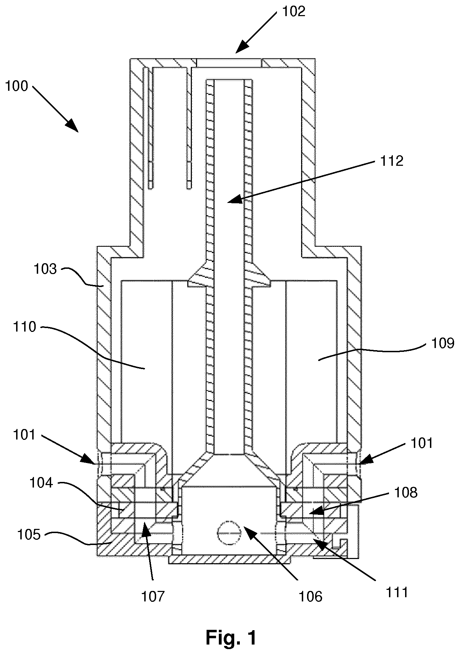

Figs. 1 to 7 illustrate a dry powder drug inhaler 100. The dry powder drug inhaler 100 comprises air inlets 101 and an air outlet 102. The outlet 102 is arranged at a first end of the dry powder drug inhaler 100, while the inlets 101 is arranged at a zone in an opposite second end of the dry powder drug inhaler 100. The outlet 102 is arranged centrally, along the longitudinal axis of the dry powder drug inhaler 100. The inlets 101 may be arranged at a radial, in relation to the longitudinal axis of the dry powder drug inhaler 100, periphery of the dry powder inhaler 100, such that the inlets 101 lead inhaled air transversally and radially towards the central portion of the dry powder inhaler 100.

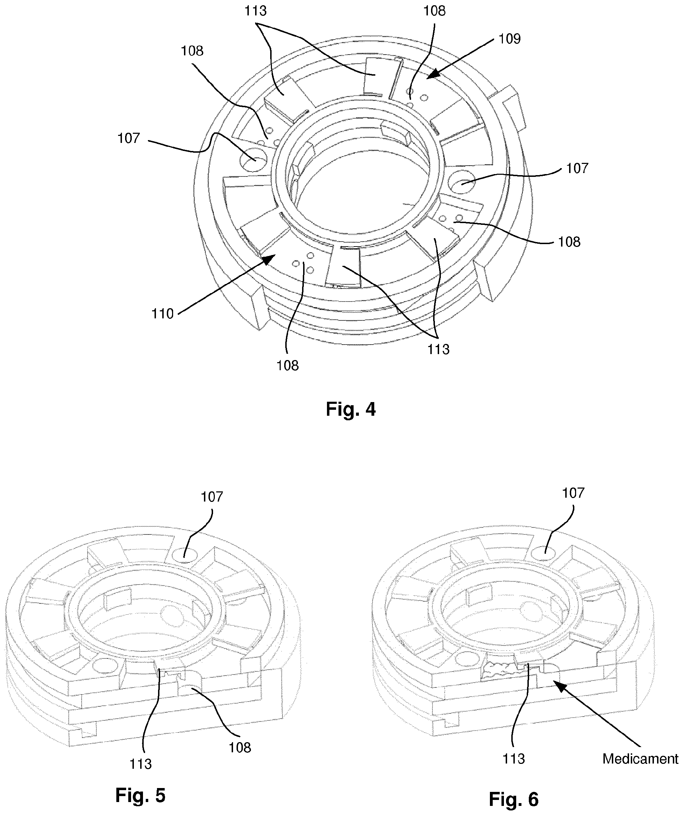

As disclosed in the embodiment according to Fig. 4, which will be further described below, the inlets 101 may however also be positioned with a direction being parallel with the central axis of the dry powder inhaler 100.

The number of inlets and outlets may be different from what is disclosed in Figs. 1 to 4. The number of inlets may for example be adjusted in accordance with needs and specific inhaler design, such that a number of smaller inlets, for reducing pressure fall over the inhaler, are arranged circumferentially of the dry powder inhaler 100. This is not shown. In a corresponding manner the number of air outlets may be adjusted in accordance with needs and specific inhaler design.

The different parts of the dry powder inhaler 100 may be manufactured in a suitable material, such as injection moldable plastics, such as thermoplastics.

The dry powder inhaler 100 comprises three major parts in form of an upper proximal reservoir housing 103, a dose disc 104, and a lower distal twister 105. The reservoir housing 103 and the twister cooperates so as to house the dose disc 104 in between these two. The twister 105 cooperates with the dose disc 104, such that the dose disc 104 may be rotated, via rotation and twisting of the twister 105, between a dose administering position and a dose collecting position. This may be accomplished by interconnecting the dose disc 104 and the twister 105 via interconnecting grooves and ribs, or letting the twister 105 extend longitudinally centrally of the dose disc 104 and connected thereto, such as disclosed for example in Fig. 1. Preferably, the rotation of the dose disc 104 has two end positions, corresponding to the dose administering position and the dose collecting position, in its relation with the reservoir housing 103, in a known manner.

In the dose administering position, the inlets 101 are in fluid communication with a mixing and deaggregation chamber 106 via dosage communications 107. The dosage communications 107 then run through openings 108 in the dose disc 104. Hence, the openings 108, in the dose administering position, is superimposed the communications 107. When rotating the dose disc 104 into a dose collecting position, the openings 108 are rotated away from fluid communication with the inlets 101 and the chamber 106. Instead, the openings 108 are rotated into medicament reservoirs 109, 110, wherein the openings 108 may collect a medicament housed in the reservoirs 109, 110. The medicament contained in the medicament reservoir 109 may be a medicament different from the medicament contained in the medicament reservoir 110. Due to the two reservoirs 109, 110, the inhaler 100 may deliver two substances in one inhalation, said two substances otherwise being incompatible, meaning that these two substances not would be possible to be comprised in one joint reservoir, such that a dry powder inhaler device 100 in which effective and satisfactory dispersion of the dry powder is obtained, which inhaler 100 can administer medicament comprising substances which can be incompatible in mixture or for other reasons are preferred to have in separate reservoirs.

It is possible to arrange the dose disc 104 and the openings 108 thereof such that when a first set of two openings 108 are superimposed the communications 107, i.e. in a dose administering position, a second set of two openings 108 are positioned in the medicament reservoirs 109, 110, respectively. Additionally, the distribution of the openings 108 on the dose disc 104 is such that the dose disc may be rotated in one direction only, which means that when the second set of two openings 108 are superimposed the communications 107, the first set of openings 108 are positioned in the medicament reservoirs 109, 110, respectively. It is also possible to rotate the dose disc 104 in a first direction to superimpose openings 108 over communications 107 in dose administering position, and then rotate the dose disc 104 in the opposite direction into the dose collecting position, to thereafter again rotate the dose disc in the first direction into the dose administering position. When the dose disc 104 is rotated in a first direction into the dose administering position and the opposite direction into the dose collecting position, the dose disc 104 may have rotational stops in the dose administering position and the dose collecting position, respectively, to ensure good superimposition over communications 107 and positioning in the medicament reservoirs 109, 110, respectively.

It is also envisioned to provide the inhaler with more than two, such as three, four, five, or six, reservoirs 109, 110, with the same arrangement of inlets, outlets, communications, dose disc, openings etc., within the ambit of the present invention.

The use of the dose disc 104 and its cooperation with the twister 105 and the reservoirs 109, 110 allows for a very cost effective solution, while simultaneously ensuring a high dose accuracy and the other benefits disclosed herein.

The dosage communications 107 are - in the embodiment disclosed in Fig. 1 - S-shaped. The S-shape is such that that the communications 107 starts at inlets 101 and extend downstream (during inhalation) in a central and transversal direction, where after they bend downwards and distally to extend in a longitudinal and distal direction to - in the dose administering position - pass through the openings 108. In this way, when medicament is positioned in the openings 108, the change of air flow direction will increase the turbulence of the air flow, which will facilitate initial deaggregation of the medicament in the openings 108. This ensures that the all the medicament in the openings 108 will follow the air flow in the communications into the chamber 106, and not partly remain in the openings 108. Downstream (again during inhalation) the openings 108, the communications 107 again bend to be directed into a central and transversal direction, to exit into the chamber 106. Distally below the openings 108, when the communications 107 bend centrally and transversally, ledges 111 are formed. These ledges 111 ensures that medicament intended to be held in the openings 108 until inhalation, which medicament may fall down, still may follow the inhaled air into the chamber 106. This means for example that the reservoirs 109, 110 may comprise a dry powder medicament in form of both a dry powder medicament in form of a micronized formulation or a carrier based formulation, or mixtures thereof. The inhaler 100 may then for example comprise a dry powder medicament in form of a micronized formulation in the first reservoir 109 and a dry powder medicament in form of a carrier based formulation in the second reservoir 110.

The directions of the two communications 107 when entering chamber 106 are arranged such that the air flows, and hence medicament flows during inhalation, when the inhaler 100 is in a dose administering position, are intersecting, such as to cross each other or coincide with each other. The flow directions from the communications 107 will coincide with each other when they are arranged on opposing sides of the chamber 106. The flow directions from the communications will cross each other when these directions are angled in relation to each other but when both are directed towards the central longitudinal axis of the inhaler 100. In this way, the medicament in the medicament flows will physically interact to increase deaggregation of the medicaments, which may increase dose uniformity, since the need for deflectors then is decreased. This feature also adds the possibility to combine or adapt the inhaler 100 for deliverance of micronized formulations and/or carrier based formulation. Of course, it also possible to combine the feature of crossing or coinciding flows from the two communications 107 with deflectors, even though the need thereof is decreased.

Depending on the medicament to be administered, and the formulation thereof, the openings 108 may be more than one opening 108 per communication 107, such as a set of openings 108, as disclosed in Fig. 4. Some medicaments have other aggregation characteristics, making it difficult to retain the medicament as a "plug" in the opening 108. Then, it may be preferable to make several openings 108 with a relatively smaller diameter. Alternatively, this can be to deliver a smaller amount of powder. This feature also adds the possibility to combine or adapt the inhaler 100 for deliverance of micronized formulations and/or carrier based formulation.

During inhalation, the medicament will then follow the air flow through the communications 107, into the chamber 106, wherein the air/medicament streams from the different communications 107 will cross, such that the medicament agglomerates will collide to increase deaggregation, where after a jet stream of finely dispersed medicament and air will continue through an inhalation chimney 112 out of the inhalator 100 through outlet 102, into the lungs of the user. The chimney 112 increases jet formation, allowing for a maintained low aggregation of medicament, hence increasing potential of medicament to reach out far in the lungs of the patient. The chimney is generally tubular, but could optionally be provided with deflectors, to further increase jet creation. Such deflectors could be bumps or spiral-shaped ridges, extending along the length of the chimney from the chamber 106 to the outlet 102. The chimney 112 does not necessarily have to be directed upwardly; it can just as well be directed downwardly or to the sides, whereby the outlet 102 naturally instead also is positioned at the bottom or on the sides, respectively. Additionally, the chimney 112 does not have to be generally tubular, but could be bent or sinus-shaped, depending on where on the inhaler 100 it is preferred to position the outlet 112. For flow characteristics and dose reliability and maintenance, it is however preferred to have it directed upwardly and generally tubular with optional deflectors. The general shape of the chimney 112 may also be such as to have differences in cross-sectional area, such as cone-shaped. In this way, the flow velocity in the chimney may be regulated, so as to help in deaggregation at chosen parts.

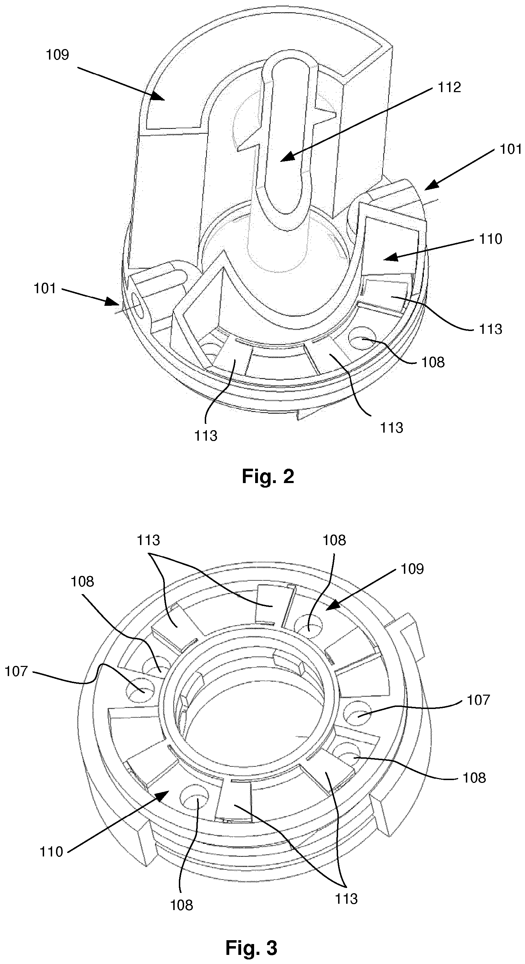

As disclosed in Figs. 2 to 7, the reservoirs 109, 110 could be provided with medicament scrapers 113. The scrapers 113 are suspended at the bottom of the reservoirs 109, 110, such that they bear upon the dose disc 104. The scrapers will pass over the openings 108 of the dose disc 104, so that excessive medicament is removed from the openings 108, to ensure correct dose volume. Also, the scrapers 113 will aid in compacting medicament in the openings 108, which will improve retention of medicament in openings 108 when the dose disc has been rotated into the dose administering position. Since the scrapers 113 are suspended on the reservoirs 109, 110, they will automatically slide along the upper proximal surface of the dose disc 104, when the dose disc 104 is rotated between the dose administering position and dose collecting position. Preferably, each reservoir 109, 110 have a number of scrapers 113 evenly distributed along the bottom of the reservoirs 109, 110. In this way, the scrapers 113 do not only aid in obtaining correct dose volume and dose compacting, but also aid in distributing medicament at the bottom of the reservoirs 109, 110. The number of scrapers 113 per reservoir 109, 110 could for example be selected in the interval of 1 to 6, such as 2 to 4, such as 3. It is also envisioned that the scrapers 113 are arranged in an uneven distribution in the reservoirs 109, 110, if certain reservoirs are configured such that an uneven distribution of the scrapers 113 will have a beneficial effect on the medicament distribution along the bottom of the reservoirs 109, 110.

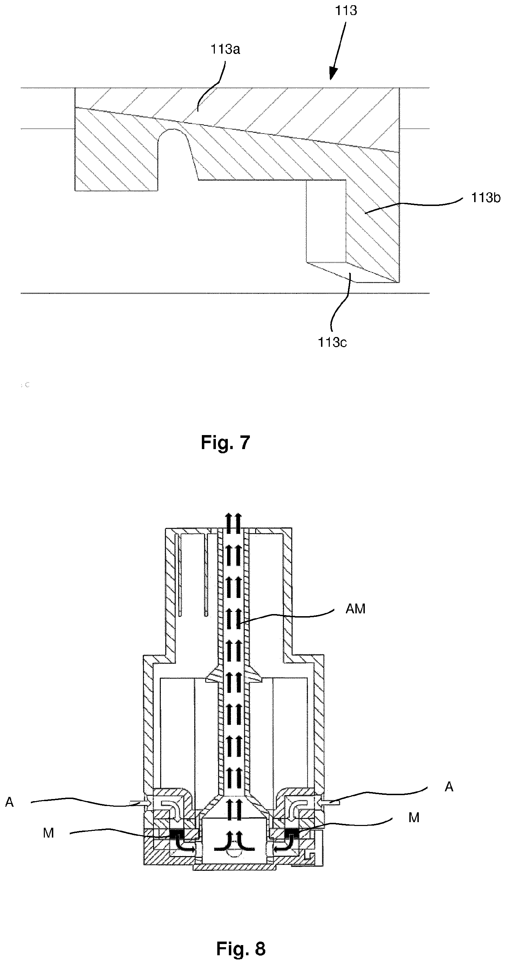

As disclosed in Fig. 7, being a close up cross sectional view of a scraper 113, the scraper 113 could comprise a scraper base portion 113a and a scraper tip portion 113b. The scraper base portion 113a is suspended on the reservoirs 109, 110. The scraper tip portion 113b then extends distally from the scraper base portion 113a. To increase efficiency of the scraper 113 with regard to scraping action on the dose disc 104, the base portion 113a could be manufactured of a resilient material. Such a resilient material could for example be a rubber material. The scraper tip portion 113b is however preferably made of a plastic material, such as a thermoplastic material, of the same kind as the rest of the inhaler 100, such as the dose disc 104, to allow for improved force attributing characteristics and improved interaction with the edge of the opening 108. The scraper tip portion 113b may be provided with a slanted tip 113c. The slanted tip 113c further improves interaction with the edge of the opening 108.

During use, the user will then simply rotate the dose disc 104 in one direction into a dose collecting position if the dose disc is in a dose administering position. Thereafter, the dose disc 104 is rotated preferably into the opposite direction to reach the dose administering position. If the dose disc 104 is already in the dose collecting position, of course the first rotation into the dose collecting position may of course be omitted. During these rotations, the scraper 113 will fill the openings 108 of the dose disc 104 in the reservoirs 109, 110 - the scraper 113 aiding the filling when rotated in both directions. After the dose disc 104 has been rotated into the dose administering position, the openings 108 are filled with medicament - optionally two different medicaments - and in fluid communication with the communications 107. Then the user puts his/her mouth at outlet 102 and inhales. During inhalation air A will enter the inhaler 100 through inlets 101 and flow through communications 107 to carry therewith the medicament(s) M in the openings 108, in accordance with Fig. 8. The air/medicament flow AM will then enter the chamber 106. In the chamber 106, the air/medicament flows AM from the communications will cross each other, such that deaggregation of the medicaments M will increase. Also, the flow characteristics, such as jet stream formation, will increase. Thereafter, the air/medicament flow AM - now comprising air/medicament flows from both communications 107, will go up through the inhaler chimney 112 and out into the lungs of the user through outlet 102.

Although, the present invention has been described above with reference to specific embodiments, it is not intended to be limited to the specific form set forth herein. Rather, the invention is limited only by the accompanying claims.

In the claims, the term "comprises/comprising" does not exclude the presence of other elements or steps. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A dry powder inhaler (100) with a distal end and a proximal end, the dry powder inhaler comprising:
at least one inlet (101) and at least one outlet (102), wherein a communication between said at least one inlet (101) and said at least one outlet (102) at least comprises a mixing and deaggregation chamber (106) and at least two dosage communications (107) between the at least one inlet (101) and the chamber (106), wherein the at least one outlet (102) is arranged at a first end of the dry powder drug inhaler (100), while the at least one inlet (101) is arranged at a zone in an opposite second end of the dry powder inhaler (100), wherein the first end is the proximal end and the opposite, second end is the distal end;
a first medicament reservoir (109) for housing a dry powder first medicament;
a second medicament reservoir (110) for housing a dry powder second medicament;
a dosage mechanism (104, 108) for arranging at least one dose of a medicament between the at least one inlet (101) and the chamber (106), such that said at least one dose may be delivered upon inhalation at said at least one outlet (102) through said communications (107);
wherein the dosage mechanism (104, 108) is configured to withdraw an inhaler metered dose of the dry powder in the first and second medicament reservoirs (109, 110) in a dose collecting position from the reservoirs (109, 110) into said dosage communications (107) in a dose administering position;
wherein the dosage mechanism (104, 108) comprises a dose disc (104) with at least one opening (108) per reservoir (109, 110), wherein the dose disc (104) may be rotated between the dose collecting position, wherein the openings (108) are positioned in the reservoirs (109, 110), and the dose administering position, wherein the openings (108) communicate with said dosage communications (107);
wherein said dosage communications (107) have directions when entering the chamber (106) for allowing jet streams through communications (107) to interact, such that medicament in said jet streams can interact to deaggregate;
**characterised in that** said dosage communications (107) have intersecting directions when entering the chamber (106).

2. The inhaler (100) according to claim 1, wherein said dosage communications (107) are opposing each other, such that they have coinciding directions when entering the chamber (106).

3. The inhaler (100) according to claim 1, further comprising one dosage communication (107) per reservoir (109, 110), said dosage communications (107) extending from one inlet (101) each to the mixing and deaggregation chamber (106).

4. The inhaler according to claim 1 or 3, wherein the dosage communications (107) each comprise a ledge (111) distally of respective opening (108) in the dose administering position.

5. The inhaler according to any of claims 1, 3, or 4, wherein the dosage communications (107) are S-shaped between the at least one inlet (101) and the chamber (106).

6. The inhaler (100) according to any of claims 1, or 3 to 5, comprising more than one opening (108) per dosage communication (107).

7. The inhaler (100) according to any of claims 1, or 3 to 6, comprising an inhalation chimney (112) interconnecting the chamber (106) and the at least one outlet (102).

8. The inhaler (100) according to claim 7, wherein the chimney (112) comprises deflectors.

9. The inhaler (100) according to any of claims 1, or 3 to 8, wherein at least one medicament scraper (113) is suspended in each reservoir (109, 110), such that the scraper (113) bear upon the dose disc (104).

10. The inhaler (100) according to claim 9, wherein the number of scrapers (113) per reservoir (109, 110) is selected in the interval from 1 to 6.

11. The inhaler (100) according to claim 9 or 10, wherein the at least one medicament scraper (113) comprises a scraper base portion (113a) and a scraper tip portion (113b).

12. The inhaler (100) according to claim 11, wherein the scraper base portion (113a) is of a resilient material.

13. The inhaler (100) according to claim 12, wherein the resilient material is a rubber material.

14. The inhaler (100) according to any of claims 1, or 3 to 12, wherein the first (109) and second reservoir (110) comprise a dry powder medicament in form of a micronized formulation or a carrier based formulation, or mixtures thereof.

15. The inhaler (100) according to any of claims 1, or 3 to 12, wherein the first reservoir (109) comprises a dry powder medicament in form of a micronized formulation and the second reservoir (110) comprises a dry powder medicament in form of a carrier based formulation.

## Patentansprüche

1. Ein Trockenpulverinhalator (100) mit einem distalen Ende und einem proximalen Ende, wobei der Trockenpulverinhalator umfasst:
mindestens einen Einlass (101) und mindestens einen Auslass (102), wobei eine Verbindung zwischen dem mindestens einen Einlass (101) und dem mindestens einen Auslass (102) mindestens eine Misch- und Deaggregationskammer (106) und mindestens zwei Dosierungsverbindungen (107) zwischen dem mindestens einen Einlass (101) und der Kammer (106) umfasst, wobei der mindestens eine Auslass (102) an einem ersten Ende des Trockenpulver-Arzneimittelinhalators (100) angeordnet ist, während der mindestens eine Einlass (101) an einer Zone in einem gegenüberliegenden zweiten Ende des Trockenpulverinhalators (100) angeordnet ist, wobei das erste Ende das proximale Ende und das gegenüberliegende, zweite Ende das distale Ende ist,
einen ersten Arzneimittelbehälter (109) zur Aufnahme eines ersten Trockenpulver-Medikaments,
einen zweiten Arzneimittelbehälter (110) zur Aufnahme eines zweiten Trockenpulver-Medikaments,
einen Dosierungsmechanismus (104, 108) zum Anordnen mindestens einer Dosis eines Medikaments zwischen dem mindestens einen Einlass (101) und der Kammer (106), so dass die mindestens eine Dosis bei Inhalation an dem mindestens einen Auslass (102) durch die Verbindungen (107) abgegeben werden kann,
wobei der Dosierungsmechanismus (104, 108) so konfiguriert ist, dass er eine im Inhalator abgemessene Dosis des Trockenpulvers in den ersten und zweiten Medikamentenreservoirs (109,110) in einer Dosis-Sammelposition aus den Reservoirs (109,110) in die Dosierungsverbindungen (107) in einer Dosis-Verabreichungsposition abzieht,
wobei der Dosierungsmechanismus (104, 108) eine Dosisscheibe (104) mit mindestens einer Öffnung (108) pro Reservoir (109,110) umfasst, wobei die Dosisscheibe (104) zwischen der Dosis-Sammelposition, in der die Öffnungen (108) in den Reservoirs (109,110) angeordnet sind, und der Dosis-Verabreichungsposition, in der die Öffnungen (108) mit den Dosierungsverbindungen (107) kommunizieren, rotiert werden kann,
wobei die Dosierungsverbindungen (107) Richtungen haben, wenn sie in die Kammer (106) eintreten, um eine Wechselwirkung von Strahlströmen durch Verbindungen (107) zu ermöglichen, so dass das Medikament in den Strahlströmen interagieren kann, um sich zu deaggregieren,
**dadurch gekennzeichnet, dass** die Dosierungsverbindungen (107) sich schneidende Richtungen haben, wenn sie in die Kammer (106) eintreten.

2. Der Inhalator (100) nach Anspruch 1, wobei die Dosierungsverbindungen (107) einander entgegengesetzt sind, so dass sie beim Eintreten in die Kammer (106) übereinstimmende Richtungen haben.

3. Der Inhalator (100) nach Anspruch 1, ferner mit einer Dosierungsverbindung (107) pro Reservoir (109, 110), wobei sich die Dosierungsverbindungen (107) von einem Einlass (101) jeweils zu der Misch- und Deaggregationskammer (106) erstrecken.

4. Der Inhalator nach Anspruch 1 oder 3, wobei die Dosierungsverbindungen (107) jeweils eine Leiste (111) distal der jeweiligen Öffnung (108) in der Dosis-Verabreichungsposition umfassen.

5. Der Inhalator nach einem der Ansprüche 1, 3 oder 4, wobei die Dosierungsverbindungen (107) zwischen dem mindestens einen Einlass (101) und der Kammer (106) S-förmig sind.

6. Der Inhalator (100) nach einem der Ansprüche 1 oder 3 bis 5, der mehr als eine Öffnung (108) pro Dosierungsmitteilung (107) aufweist.

7. Der Inhalator (100) nach einem der Ansprüche 1 oder 3 bis 6, bestehend aus einem Inhalationskamin (112), der die Kammer (106) und den mindestens einen Auslass (102) miteinander verbindet.

8. Der Inhalator (100) nach Anspruch 7, wobei der Kamin (112) Deflektoren aufweist.

9. Der Inhalator (100) nach einem der Ansprüche 1 oder 3 bis 8, wobei mindestens ein Medikamentenschaber (113) in jedem Reservoir (109,110) aufgehängt ist, so dass der Schaber (113) auf der Dosisscheibe (104) schlägt.

10. Der Inhalator (100) nach Anspruch 9, wobei die Anzahl der Schaber (113) pro Reservoir (109,110) im Intervall von 1 bis 6 gewählt ist.

11. Der Inhalator (100) nach Anspruch 9 oder 10, wobei der mindestens eine Medikamentenschaber (113) einen Schaber-Basisabschnitt (113a) und einen Schaber-End- oder Spitzenabschnitt (113b) umfasst.

12. Der Inhalator (100) nach Anspruch 11, bei dem der Schaber-Basisabschnitt (113a) aus einem elastischen Material gemacht ist.

13. Der Inhalator (100) nach Anspruch 12, wobei das elastische Material ein Gummimaterial ist.

14. Der Inhalator (100) nach einem der Ansprüche 1 oder 3 bis 12, wobei das erste (109) und das zweite Reservoir (110) ein Trockenpulver-Medikament in Form einer mikronisierten Formulierung oder einer Formulierung auf Trägerbasis oder Mischungen davon umfassen.

15. Der Inhalator (100) nach einem der Ansprüche 1 oder 3 bis 12, wobei das erste Reservoir (109) ein Trockenpulver-Medikament in Form einer mikronisierten Formulierung und das zweite Reservoir (110) ein Trockenpulver-Medikament in Form einer Formulierung auf Trägerbasis umfasst.

## Revendications

1. Inhalateur (100) de poudre sèche avec une extrémité distale et une extrémité proximale, l'inhalateur de poudre sèche comprenant :
au moins un orifice d'admission (101) et au moins un orifice d'évacuation (102), dans lequel une communication entre ledit au moins un orifice d'admission (101) et ledit au moins un orifice d'évacuation (102) comprend au moins une chambre (106) de mélange et de désagrégation et au moins deux communications (107) de dosage entre l'au moins un orifice d'admission (101) et la chambre (106), dans lequel l'au moins un orifice d'évacuation (102) est agencé au niveau d'une première extrémité de l'inhalateur (100) de médicament en poudre sèche, alors que l'au moins un orifice d'admission (101) est agencé au niveau d'une zone dans une seconde extrémité opposée de l'inhalateur (100) de poudre sèche, dans lequel la première extrémité est l'extrémité proximale et la seconde extrémité opposée est l'extrémité distale ;
un premier réservoir (109) de médicament pour contenir un premier médicament en poudre sèche ;
un second réservoir (110) de médicament pour contenir un second médicament en poudre sèche ;
un mécanisme de dosage (104, 108) pour agencer au moins une dose d'un médicament entre l'au moins un orifice d'admission (101) et la chambre (106), de telle sorte que ladite au moins une dose puisse être délivrée lors de l'inhalation au niveau dudit au moins un orifice d'évacuation (102) à travers lesdites communications (107) ;
dans lequel le mécanisme de dosage (104, 108) est configuré pour prélever une dose mesurée par l'inhalateur de la poudre sèche dans les premier et second réservoirs (109, 110) de médicament dans une position de collecte de dose à partir des réservoirs (109, 110) dans lesdites communications (107) de dosage dans une position d'administration de dose ;
dans lequel le mécanisme de dosage (104, 108) comprend un disque de doses (104) avec au moins une ouverture (108) par réservoir (109, 110), dans lequel le disque de doses (104) peut pivoter entre la position de collecte de dose, dans laquelle les ouvertures (108) sont positionnées dans les réservoirs (109, 110) et la position d'administration de dose, dans laquelle les ouvertures (108) communiquent avec lesdites communications (107) de dosage ;
dans lequel lesdites communications (107) de dosage ont des directions lorsqu'elles entrent dans la chambre (106) pour permettre l'interaction de flux d'éjection par des communications (107), de telle sorte que le médicament dans lesdits flux d'éjection puisse interagir pour se désagréger ;
**caractérisé en ce que** lesdites communications (107) de dosage ont des directions se croisant lorsqu'elles entrent dans la chambre (106).

2. Inhalateur (100) selon la revendication 1, dans lequel lesdites communications (107) de dosage sont opposées les unes aux autres, de telle sorte qu'elles ont des directions coïncidentes lorsqu'elles entrent dans la chambre (106).

3. Inhalateur (100) selon la revendication 1, comprenant en outre une communication (107) de dosage par réservoir (109, 110), lesdites communications (107) de dosage s'étendant à partir d'un orifice d'admission (101) chacune jusqu'à la chambre (106) de mélange et de désagrégation.

4. Inhalateur selon la revendication 1 ou 3, dans lequel les communications (107) de dosage comprennent chacune un rebord (111) distalement de l'ouverture (108) respective dans la position d'administration de dose.

5. Inhalateur selon l'une quelconque des revendications 1, 3, ou 4, dans lequel les communications (107) de dosage sont en forme de S entre l'au moins un orifice d'admission (101) et la chambre (106) .

6. Inhalateur (100) selon l'une quelconque des revendications 1, ou 3 à 5, comprenant plus d'une ouverture (108) par communication (107) de dosage.

7. Inhalateur (100) selon l'une quelconque des revendications 1, ou 3 à 6, comprenant une cheminée (112) d'inhalation reliant la chambre (106) et l'au moins un orifice d'évacuation (102).

8. Inhalateur (100) selon la revendication 7, dans lequel la cheminée (112) comprend des déflecteurs.

9. Inhalateur (100) selon l'une quelconque des revendications 1, ou 3 à 8, dans lequel l'au moins un racloir (113) à médicament est suspendu dans chaque réservoir (109, 110), de telle sorte que le racloir (113) repose sur le disque de doses (104).

10. Inhalateur (100) selon la revendication 9, dans lequel le nombre de racloirs (113) par réservoir (109, 110) est sélectionné dans l'intervalle de 1 à 6.

11. Inhalateur (100) selon la revendication 9 ou 10, dans lequel l'au moins un racloir (113) à médicament comprend une partie base de racloir (113a) et une partie pointe de racloir (113b).

12. Inhalateur (100) selon la revendication 11, dans lequel la partie base de racloir (113a) est en un matériau élastique.

13. Inhalateur (100) selon la revendication 12, dans lequel le matériau élastique est un matériau de caoutchouc.

14. Inhalateur (100) selon l'une quelconque des revendications 1, ou 3 à 12, dans lequel les premier (109) et second réservoirs (110) comprennent un médicament en poudre sèche sous la forme d'une formulation micronisée ou d'une formulation basée sur un véhicule, ou des mélanges de celles-ci.

15. Inhalateur (100) selon l'une quelconque des revendications 1, ou 3 à 12, dans lequel le premier réservoir (109) comprend un médicament en poudre sèche sous la forme d'une formulation micronisée et le second réservoir (110) comprend un médicament en poudre sèche sous la forme d'une formulation basée sur un véhicule.
